# EUROPEAN PATENT SPECIFICATION

(11) **EP 1 728 466 B1**
(45) Date of publication and mention of the grant of the patent: **14.01.2009**
(21) Application number: 06252780.9
(22) Date of filing: 30.05.2006
(51) Int. Cl.: A61B 1/12, F16L 33/16, A61B 1/00

(54) **Endoscope reprocessor connectors having reduced occlusion**
Anschlüsse für ein Endoskopreinigungsgerät mit verminderter Verschlusstendenz
Dispositif de retraitement pour endoscopes comportant une tendance de bouchage reduite

(30) Priority: 31.05.2005 US 141431
(43) Date of publication of application: 06.12.2006
(73) Proprietor: ETHICON, INC., Somerville, New Jersey 08876 (US)
(72) Inventor: Nhuyen, Nick Ngoc, Silverado, CA 92676 (US); Morrison, Todd, Dana Point, CA 92629 (US)
(74) Representative: Fisher, Adrian John

(56) References cited:
- EP-B1- 0 071 058
- EP-B1- 1 253 849
- US-A- 5 833 935
- US-A- 5 840 251
- US-A- 6 041 794

## Description

### BACKGROUND OF THE INVENTION

The present invention relates to endoscope reprocessors and to connectors therefore. Specifically connectors which reduce seclusion between the endoscope and the connector.

Endoscopes and similar medical devices having channels or lumens formed therethrough are being used on an ever increasing basis in the performance of medical procedures. The popularity of these devices has led to calls for improvements in the decontamination of these devices between use, both in terms of the speed of the decontamination and the effectiveness of the decontamination.

One popular method for cleaning and disinfection or sterilization of such endoscopes employs an automated endoscope reprocessor which both washes and then disinfects or sterilizes the endoscope. Typically such a unit comprises a basin with a selectively opened and closed cover member to provide access to the basin. Pumps connect to various channels through the endoscope to flow fluid therethrough and an additional pump flows fluid over the exterior surfaces of the endoscope. Typically, a detergent washing cycle is followed by rinsing and then a sterilization or disinfection cycle and rinse. Various connections must be made to the endoscope to achieve flow through its channels. Contact between the endoscope and a connector can lead to an occlusion. Typical procedure calls for these surfaces to be manually cleaned and swabbed with sterilization fluid prior to the connection being made, nevertheless it would be desirable to treat these areas during the endoscope reprocessing cycle.

The Lin et al. U.S. Patent No. 6,041,794 describes a connector for such use which avoids occlusion. A spring actuated surface moves away from the connection under increased fluid pressure to provide fluid contact to the surface of the endoscope at the point of connection.

U.S. Patent Nos. 6,485,684, 6,585,943, 5,795,403 and 5,833,935, disclose connectors which loosely fit onto the endoscope to allow a leakage of flow past the connector.

EP 0071058 discloses a connector of the type set forth in the preamble of the accompanying claim 1 and a method of the type set forth in the preamble of the accompanying claim 10.

### SUMMARY OF THE INVENTION

A connector according to the present invention connects a lumen in a lumen device to a source of fluid in an endoscope reprocessor. The connector comprises a coupling configured to engage with a port connected to the lumen on the lumen, the port including a first sealing surface. A flow passage goes through the connector. A sealing portion, formed of resilient material, movably connects with the flow passage and includes a second sealing surface shaped to engage the first sealing surface. The sealing portion is adapted to engage with the first sealing surface under a condition of a first flow in the connector and is further adapted to disengage from the first sealing surface under a condition of a second flow, different from the first flow, in the connector. When disengaged the fluid of the flow, such as a washing fluid or a disinfecting or sterilizing fluid, can contact the first sealing surface on the port.

In one aspect of the invention, the resilient material of the sealing portion biases the second sealing surface into contact with the first sealing surface when engaged therewith, the biasing being such that the second sealing surface seals against the first sealing surface when pressure within the flow passage associated with the first flow is below a predetermined level thereby preventing leakage of the flow there past. When pressure within the flow passage associated with the second flow is above the predetermined level the second surface does not seal against the first surface thereby allowing flow over the first sealing surface.

In a different aspect of the invention, the resilient material of the sealing portion biases the second sealing surface away from contact with the first sealing surface when engaged therewith, the biasing being such that the second sealing surface does not seal against the first sealing surface when pressure within the flow passage associated with the first flow is below a predetermined level thereby allowing flow over the first sealing surface. The sealing portion is oriented such that when pressure within the flow passage associated with the second flow is above the predetermined level such pressure urges the second sealing surface into contact with the first sealing surface thereby preventing leakage of the flow there past.

Preferably, the sealing portion is formed integral with the connector. The first sealing surface can be cylindrical with the sealing portion comprises an annular flange sized to seat against the first sealing surface. Preferably, the sealing portion comprises a free distal edge. One good material for the sealing portion is silicone.

Preferably, the first flow is associated with a pressure at the sealing portion less than 0.83 bar (12 psig) and the second flow is associated with a pressure at the sealing portion greater than 0.83 bar (12 psig). Alternatively, the first flow is associated with a pressure at the sealing portion greater than 0.35 bar (5 psig) and the second flow is associated with a pressure at the sealing portion less than 0.35 bar (5 psig).

A method according to the present invention connects a port on a lumen device to an endoscope reprocessor and flows fluid through the port. The method comprising the steps of: attaching a coupling on the endoscope reprocessor to the port, the coupling having a flow passage therethrough and a sealing portion movably attached to the flow passage, the sealing portion being moveable from a first position to a second position, and the sealing portion being formed of a resilient material biasing it into one of the first position or second position; flowing a first flow through the flow passage and into the port causing the sealing portion to move into the first position; flowing a second, different, flow through the flow passage causing the sealing portion to move into the second position; and wherein one of the first position and the second position comprises a sealing position in which the sealing portion seals against a first sealing surface on the port to prevent flow there past, and wherein the other of the first position and the second position comprises a non-sealing position in which the sealing portion is away from the first sealing surface to allow flow over the first sealing surface.

In one aspect of the invention the first position is the sealing position. The sealing portion can be biased toward the first position. In one aspect of the invention, the second flow has a higher pressure than the first flow. This can be created by increasing volume of the second flow. The higher pressure can be from about 0.35 to 0.83 bar (5 to 12 psig).

In another aspect of the invention, the second flow is in a different direction than the first flow.

The flows can be of a washing fluid, an antimicrobial agent, or other flows in an endoscope reprocessor.

### BRIEF DESCRIPTION OF THE DRAWINGS

The invention may take form in various components and arrangements of components and in various steps and arrangements of steps. The drawings are for purposes of illustrating preferred embodiments only, and are not to be construed as limiting the invention.
FIG. 1 is a front elevational view of a decontamination apparatus in accordance with the present invention;
FIG. 2 is a diagrammatic illustration of the decontamination apparatus shown in FIG. 1, with only a single decontamination basin shown for clarity;
FIG. 3 is a cut-away view of an endoscope suitable for processing in the decontamination apparatus of FIG. 1;
FIG. 4 is a cut-away view of a connector according to the present invention for connecting to the endoscope of FIG. 3;
FIG. 5 is a cut-away view of an alternative connector according to the present invention for connecting to the endoscope of FIG. 3;
FIG. 6 is a cut-away view of an alternative connector according to the present invention for connecting to the endoscope of FIG. 3;
FIG. 7 is a cut-away view of an alternative connector according to the present invention for connecting to the endoscope of FIG. 3;
FIG. 8 is a cut-away view of a channel connector according to the present invention for use in the endoscope of FIG. 3;
FIG. 9 is a cut-away view of a channel connector and separator according to the present invention for use in the endoscope of FIG. 3.
FIG. 10 is a cut-away view of an alternative connector according to the present invention for connecting to the endoscope of FIG. 3; and
FIG. 11 is a cut-away view of an alternative connector according to the present invention for connecting to the endoscope of FIG. 3.

### DETAILED DESCRIPTION OF THE PREFERRED EMBODIMENT

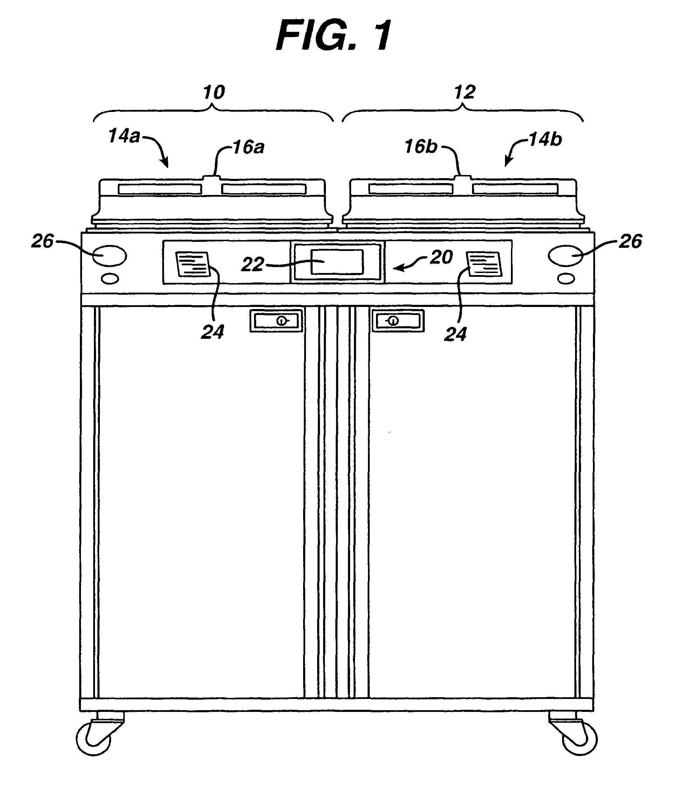

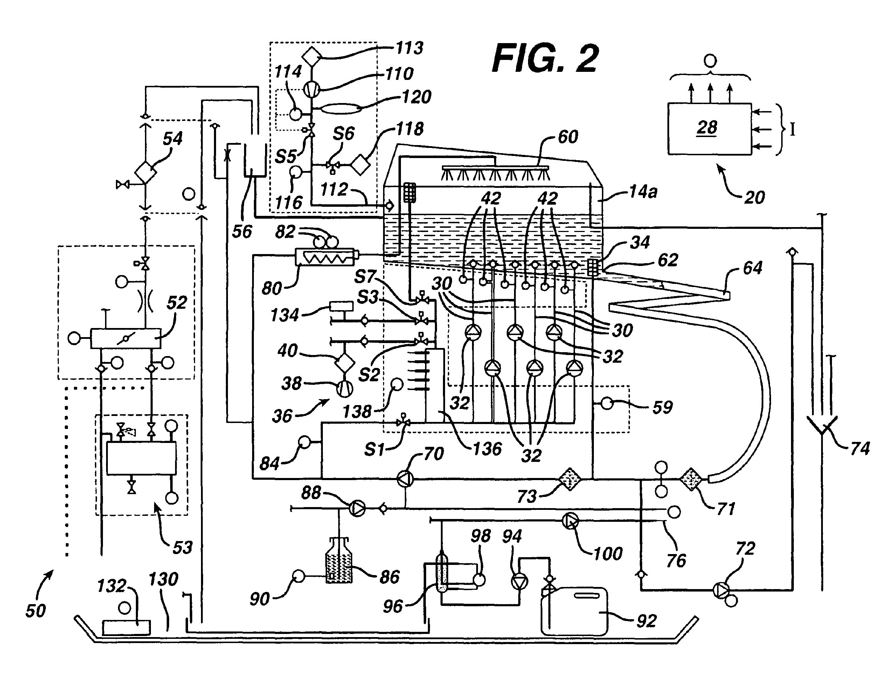

FIG. 1 shows a decontamination apparatus for decontaminating endoscopes and other medical devices which include channels or lumens formed therethrough; FIG. 2 shows the apparatus in block diagram form. The decontamination apparatus generally includes a first station 10 and a second station 12 which are at least substantially similar in all respects to provide for the decontamination of two different medical devices simultaneously or in series. First and second decontamination basins 14a, 14b receive the contaminated devices. Each basin 14a, 14b is selectively sealed by a lid 16a, 16b, respectively, preferably in a microbe-blocking relationship to prevent the entrance of environmental microbes into the basins 14a, 14b during decontamination operations. The lids can include a microbe removal or HEPA air filter formed therein for venting.

A control system 20 includes one or more microcontrollers, such as a programmable logic controller (PLC), for controlling decontamination and user interface operations. Although one control system 20 is shown herein as controlling both decontamination stations 10, 12, those skilled in the art will recognize that each station 10, 12 can include a dedicated control system. A visual display 22 displays decontamination parameters and machine conditions for an operator and at least one printer 24 prints a hard copy output of the decontamination parameters for a record to be filed or attached to the decontaminated device or its storage packaging. The visual display 22 is preferably combined with a touch screen input device. Alternatively, a keypad or the like is provided for input of decontamination process parameters and for machine control. Other visual gauges 26 such as pressure meters and the like provide digital or analog output of decontamination or medical device leak testing data.

FIG. 2 diagrammatically illustrates one station 10 of the decontamination apparatus. Those skilled in the art will recognize that the decontamination station 12 is preferably similar in all respects to the station 10 illustrated in FIG. 2. However, the station 12 has not been shown in FIG. 2 for clarity. Further, the decontamination apparatus can be provided with a single decontamination station or multiple stations.

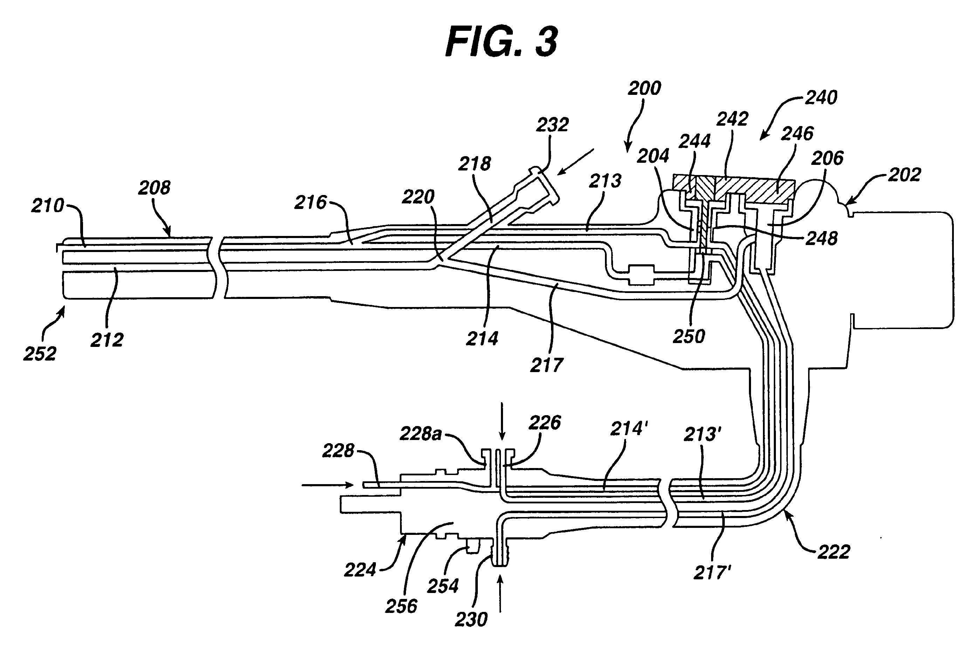

The decontamination basin 14a receives an endoscope 200 (see FIG. 3) or other medical device therein for decontamination. Any internal channels of the endoscope 200 are connected with flush lines 30. Each flush line 30 is connected to an outlet of a pump 32. The pumps 32 are preferably peristaltic pumps or the like that pump fluid, such as liquid and air, through the flush lines 30 and any internal channels of the medical device. Specifically, the pumps 32 either can draw liquid from the basin 14a through a filtered drain 34 and a first valve S1, or can draw decontaminated air from an air supply system 36 through a valve S2. The air supply system 36 includes a pump 38 and a microbe removal air filter 40 that filters microbes from an incoming air stream. It is preferable that each flush line 30 be provided with a dedicated pump 32 to ensure adequate fluid pressure and to facilitate the individual monitoring of the fluid pressure in each flush line 30. A pressure switch or sensor 42 is in fluid communication with each flush line 30 for sensing excessive pressure in the flush line. Any excessive pressure sensed is indicative of a partial or complete blockage, e.g., by bodily tissue or dried bodily fluids, in a device channel to which the relevant flush line 30 is connected. The isolation of each flush line 30 relative to the others allows the particular blocked channel to be easily identified and isolated, depending upon which sensor 42 senses excessive pressure.

The basin 14a is in fluid communication with a water source 50 such as a utility or tap water connection including hot and cold inlets and a mixing valve 52 flowing into a break tank 56. A microbe removal filter 54, such as a 0.2 µm or smaller absolute pore size filter, decontaminates the incoming water which is delivered into the break tank 56 through the air gap to prevent backflow. A pressure type level sensor 59 monitors liquid levels within the basin 14a. An optional water heater 53 can be provided if an appropriate source of hot water is not available.

The condition of the filter 54 can be monitored by directly monitoring the flow rate of water therethrough or indirectly by monitoring the basin fill time using a float switch or the like. When the flow rate drops below a select threshold, this indicates a partially clogged filter element that requires replacement.

A basin drain 62 drains liquid from the basin 14a through an enlarged helical tube 64 into which elongated portions of the endoscope 200 can be inserted. The drain 62 is in fluid communication with a recirculation pump 70 and a drain pump 72. The recirculation pump 70 recirculates liquid from the basin drain 62 to a spray nozzle assembly 60 which sprays the liquid into the basin 14a and onto the endoscope 200. Coarse and fine screens 71 and 73, respectively, filter out particles in the recirculating fluid. The drain pump 72 pumps liquid from the basin drain 62 to a utility drain 74. A level sensor 76 monitors the flow of liquid from the pump 72 to the utility drain 74. The pumps 70 and 72 can be simultaneously operated such that liquid is sprayed into the basin 14a while it is being drained to encourage the flow of residue out of the basin and off of the device. Of course, a single pump and a valve assembly could replace the dual pumps 70, 72.

An inline heater 80, with temperature sensors 82, downstream of the recirculation pump 70 heats the liquid to optimum temperatures for cleaning and disinfection. A pressure switch or sensor 84 measures pressure downstream of the circulation pump 70.

Detergent solution 86 is metered into the flow upstream of the circulation pump 70 via a metering pump 88. A float switch 90 indicates the level of detergent available. Typically, only a small amount of disinfectant 92 is required. To more accurately meter this, a dispensing pump 94 fills a pre-chamber 96 under control of a hi/low level switch 98 and of course the control system 20. A metering pump 100 meters a precise quantity of disinfectant as needed.

Endoscopes and other reusable medical devices often include a flexible outer housing or sheath surrounding the individual tubular members and the like that form the interior channels and other parts of the device. This housing defines a closed interior space, which is isolated from patient tissues and fluids during medical procedures. It is important that the sheath be maintained intact, without cuts or other holes that would allow contamination of the interior space beneath the sheath. Therefore, the decontamination apparatus includes means for testing the integrity of such a sheath.

An air pump, either the pump 38 or another pump 110, pressurizes the interior space defined by the sheath of the device through a conduit 112 and a valve S5. Preferably, a HEPA or other microbe-removing filter 113 removes microbes from the pressurizing air. An overpressure switch 114 prevents accidental over pressurization of the sheath. Upon full pressurization, the valve S5 is closed and a pressure sensor 116 looks for a drop in pressure in the conduit 112 which would indicate the escape of air through the sheath. A valve S6 selectively vents the conduit 112 and the sheath through an optional filter 118 when the testing procedure is complete. An air buffer 120 smoothes out pulsation of pressure from the air pump 110.

Preferably, each station 10 and 12 each contain a drip basin 130 and spill sensor 132 to alert the operator to potential leaks.

An alcohol supply 134 controlled by a valve S3 can supply alcohol to the channel pumps 32 after rinsing steps to assist in removing water from the endoscope channels.

Flow rates in the supply lines 30 can be monitored via the channel pumps 32 and the pressure sensors 42. The channels pumps 32 are peristaltic pumps which supply a constant flow. If one of the pressure sensors 42 detects too high a pressure the associated pump 32 cycles off. The flow rate of the pump 32 and its percentage on time provide a reasonable indication of the flow rate in an associated line 30. These flow rates are monitored during the process to check for blockages in any of the endoscope channels. Alternatively, the decay in the pressure from the time the pump 32 cycles off can also be used to estimate the flow rate, with faster decay rates being associated with higher flow rates.

A more accurate measurement of flow rate in an individual channel may be desirable to detect more subtle blockages. A metering tube 136 having a plurality of level indicating sensors 138 fluidly connects to the inputs of the channel pumps 32. One preferred sensor arrangement provides a reference connection at a low point in the metering tube and a plurality of sensors 138 arranged vertically thereabove. By passing a current from the reference point through the fluid to the sensors 138 it can be determined which sensors 138 are immersed and therefore determine the level within the metering tube 136. Other level sensing techniques can be applied here. By shutting valve S 1 and opening a vent valve S7 the channel pumps 32 draw exclusively from the metering tube. The amount of fluid being drawn can be very accurately determined based upon the sensors 138. By running each channel pump in isolation the flow therethrough can be accurately determined based upon the time and the volume of fluid emptied from the metering tube.

In addition to the input and output devices described above, all of the electrical and electromechanical devices shown are operatively connected to and controlled by the control system 20. Specifically, and without limitation, the switches and sensors 42, 59, 76, 84, 90, 98, 114, 116, 132 and 136 provide input I to the microcontroller 28 which controls the decontamination and other machine operations in accordance therewith. For example, the microcontroller 28 includes outputs O that are operatively connected to the pumps 32, 38, 70, 72, 88, 94, 100, 110, the valves S1-S7, and the heater 80 to control these devices for effective decontamination and other operations.

Turning also to FIG. 3, an endoscope 200 has a head part 202, in which openings 204 and 206 are formed, and in which , during normal use of the endoscope 200, an air/water valve and a suction valve are arranged. A flexible insertion tube 208 is attached to the head part 202, in which tube a combined air/water channel 210 and a combined suction/biopsy channel 212 are accommodated.

A separate air channel 213 and water channel 214, which at the location of a joining point 216 merge into the air/water channel 210, are arranged in the head part 202. Furthermore, a separate suction channel 217 and biopsy channel 218, which at the location of the joining point 220 merge into the suction/biopsy channel 212, are accommodated in the head part 202.

In the head part 202, the air channel 213 and the water channel 214 open into the opening 204 for the air/water valve. The suction channel 217 opens into the opening 206 for the suction valve. Furthermore, a flexible feed hose 222 connects to the head part 202 and accommodates channels 213', 214' and 217' which via the openings 204 and 206, are connected to the air channel 213, the water channel 214 and the suction channel 217, respectively. In practice, the feed hose 222 is also referred to as the light-conductor casing.

The mutually connecting channels 213 and 213', 214 and 214', 217 and 217' will be referred to below overall as the air channel 213, the water channel 214 and the suction channel 217.

A connection 226 for the air channel 213, connections 228 and 228a for the water channel 214 and a connection 230 for the suction channel 217 are arranged on the end section 224 (also referred to as the light conductor connector) of the flexible hose 222. When the connection 226 is in use, connection 228a is closed off. A connection 232 for the biopsy channel 218 is arranged on the head part 202.

A channel separator 240 is shown inserted into the openings 204 and 206. It comprises a body 242, and plug members 244 and 246 which occlude respectively openings 204 and 206. A coaxial insert 248 on the plug member 244 extends inwardly of the opening 204 and terminates in an annular flange 250 which occludes a portion of the opening 204 to separate channel 213 from channel 214. By connecting the lines 30 to the openings 226, 228, 228a, 230 and 232, liquid for cleaning and disinfection can be flowed through the endoscope channels 213, 214, 217 and 218 and out of a distal tip 252 of the endoscope 200 via channels 210 and 212. The channel separator 240 ensures that such liquid flows all the way through the endoscope 200 without leaking out of openings 204 and 206 and isolates channels 213 and 214 from each other so that each has its own independent flow path. One of skill in the art will appreciate that various endoscopes having differing arrangements of channels and openings will likely require modifications in the channel separator 240 to accommodate such differences while occluding ports in the head 202 and keeping channels separated from each other so that each channel can be flushed independently of the other channels. Otherwise a blockage in one channel might merely redirect flow to a connected unblocked channel.

A leakage port 254 on the end section 224 leads into an interior portion 256 of the endoscope 200 and is used to check for the physical integrity thereof, namely to ensure that no leakage has formed between any of the channels and the interior 256 or from the exterior to the interior 256.

Proper connection to the various endoscope channels is necessary to ensure adequate cleaning and sterilization thereof. Connections are made with a connection set (not shown) comprising a set of flexible tubes one end of which connects to ports on the endoscope reprocessor associated with the channel pumps 32. The other end has a connector adapted to engage a connection on the endoscope 200. As endoscopes vary by manufacturer and model different connection sets are generally provided to fit different endoscopes.

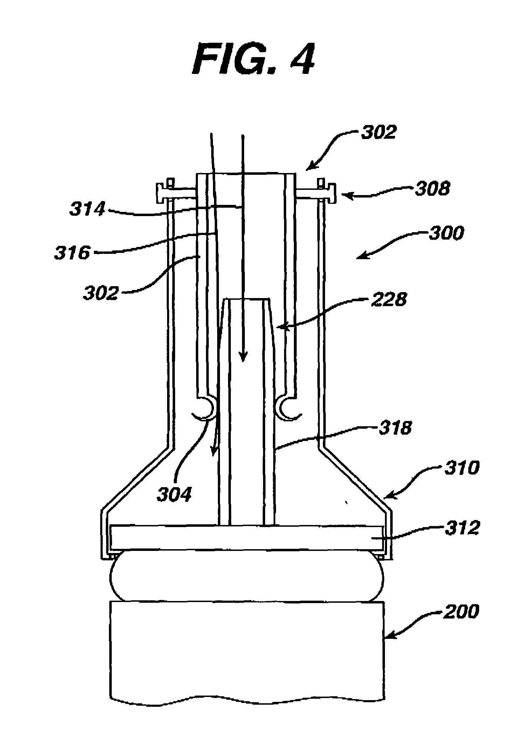

FIG. 4 shows a connector 300 for connection to connection 228 (see also FIG. 3) on the endoscope 200. A tubular body 302 terminates in a seal 304. The body and seal 302 and 304 are formed of a resilient material such as silicone or resilient plastic or polymer. The body 302 can be formed of a different material than the seal 304, and can be rigid rather than resilient. The seal 304 curves inwardly, with the curve continuing such that it ultimately flares outwardly. Preferably its thickness decreases as it continues flaring away from the body 302.

Pins 308 extend radially outwardly from the body 302. Preferably, the pins 308 are molded with the body 302. They attach to spring clips 310 which attach to a shoulder 312 on the endoscope 200 adjacent the connection 228. There are two flow paths through the connector 300. A first flow path 314 occurs under low pressure. The seal 304 engages the connection 228 and prevents fluid from escaping there passed. Therefore, the flow enters the connection 228. A second flow path 316 occurs under higher pressures. When the pressure exceeds the engaging force of the seal 304, fluid leaks past the seal 304 and it bathes an outer surface 318 of the connection 228. The spring clips 310 prevent the connector 300 from pulling off of the connection 228.

The pressure under which the seal 304 moves away from its contact with a surface on the connection 228 depends upon the size of the connection. For a large connection a pressure of 10 to 0.69 bar (10 psig) might be appropriate, whereas for a smaller connection a pressure of about 0.35 bar (5 psig) should suffice. Pressures over about 1.45 bar (21 psig) may exceed the maximum recommended pressure for most endoscopes.

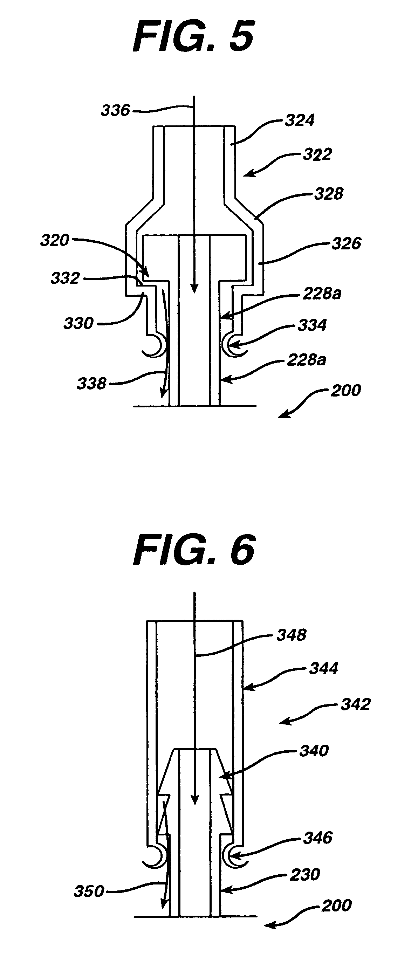

Turning also now to FIG. 5, some connections on the endoscope 200, such as the water channel connection 228a, terminate in an annular flange 320. A connector 322 connects to such a connection 228a. The connector 322 comprises a cylindrical body 324 having an expanded diameter section 326, preferably with a taper 328. A distal portion 330 of the expanded diameter section 326 extends inwardly radially to form an engaging surface 332 which abuts the flange 320 to hold the connector 322 on the connection 228a. The body 324 extends distally from the expanded diameter section 326 and terminates in a seal 334 similar to the seal 304. A low pressure flow path 336 passes through the connector 322 and into the connection 228a, with the seal 334 preventing leakage. Under higher pressure conditions, a second flow path 338 opens up as the seal 334 disengages from the connection 228a.

Turning also now to FIG. 6, other connections on the endoscope 200, such as the connection 230 take the form of a hose barb 340. A connector 342 engages the hose barb 340 on connection 230. The connector 342 comprises a cylindrical body 344 having an internal diameter slightly larger than the widest diameter of the hose barb 340. The body 344 terminates in a seal 346. The seal 346 engages the connection 230 proximally of the hose barb 340 and operates as in the previous two embodiments. Further, it engages with the hose barb 340 to prevent the connector 342 from disengaging from the connection 230. Therefore, a low pressure flow path 348 passes through the connector 342 and into the connection 230 and a higher pressure flow path 350 passes between the hose barb 340 and the body 344 and past the seal 346.

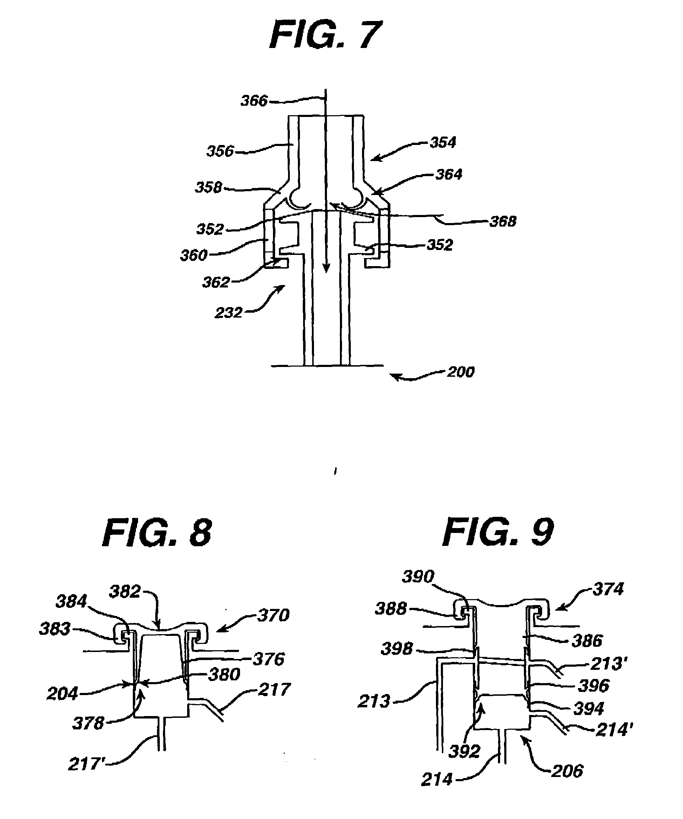

Turning also now to FIG. 7, some connections on the endoscope 200 comprise one or more outwardly extending annular flanges 352 such as on the connection 232. A connector 354 connects to the connection 232. The connector 354 comprises a body 356 which flares outwardly 358 to an expanded diameter section 360 which enhances access of fluids to the flanges 352. The expanded diameter section 360 terminates distally with an inwardly extending annular flange 362. A seal 364 extends inwardly toward the expanded diameter section 360 from the flare 358. The flange 362 on the connector 354 engages the flanges 352 on the connection 232 to hold the connector 354 on the connection 232. A first flow path 366 passes from the connector 354 into the connection 232. This flow path will be maintained even under higher pressures due to the direction in which the seal 364 flares from the body 356. Rather than provide a second flow path by changing pressure, here, flow is reversed to provide a reversed flow path 368. These parts are submersed in the same liquid which flows through the connector 354 so when flow is reversed, some of the fluid surrounding the connector 354 is drawn in to the connector 354 past the seal 364 thus contacting remaining surfaces on the connection 232. A second seal (not shown) could be provided on the connector 354 at the flange 362 and oriented to allow reverse flow.

The channel connector 240 provides another possible source of occlusion. FIGS. 8 and 9 shows a non-occluding alternative. Rather than being formed as a one-piece unit like the channel separator 240, it comprises an air/water valve channel connector 370 for insertion into the opening 204 for the air/water valve and a suction valve channel separator and connector 374 for insertion into the opening 206 for the suction valve. A one-piece unit would work as well. The air/water valve channel connector 370 comprises a cylindrical body 376 open at a distal end 378 thereof. A seal 380 is located at the distal end 378 and extends toward the interior surface of the opening 204. Unlike the seals in the prior embodiments, this seal 380 is smaller in circumference when relaxed than the circumference of the opening 204 and is thus out of engagement with the surface 204 at first. A proximal end 382 of the body 376 is closed and extends outwardly radially, downwardly and then inwardly to form an annular lip 383 which engages an annular outwardly extending flange 384 about the opening 204. In normal operation fluid will flow in through channel 217' past the seal 380 bathing the surfaces of the opening 204. Fluid can also enter through the opening 204 as these parts are submerged in fluid. As flow is increased it forces the seal 380 against the surface of the opening 204 as depicted in FIG. 8. Fluid then flows out through the suction channel 217.

The suction valve channel connector and separator 374 comprises a body 386 having a similar proximal end construction to the air/water valve channel connector 370 being closed and terminating in a lip 388 for engagement with a flange 390 about the opening 206. At a distal end 392 of the body 386, a first seal 394 extends outwardly distally and a second seal 396 extends outwardly and proximally. The seals 394 and 396 are disposed within the opening 206 outwardly of where channel 214' and the water channel 214 meet with the opening 206. A third seal 398 flares outwardly and distally from the body 386 and is disposed outwardly of where the channel 213' and the air channel 213 intersect with the opening 206. As with the channel connector 370, the seals 394, 396 and 398 do not engage the surface of the opening 206 when relaxed. They expand as fluid is flowed into the opening 206 from lines 213' and 214'.

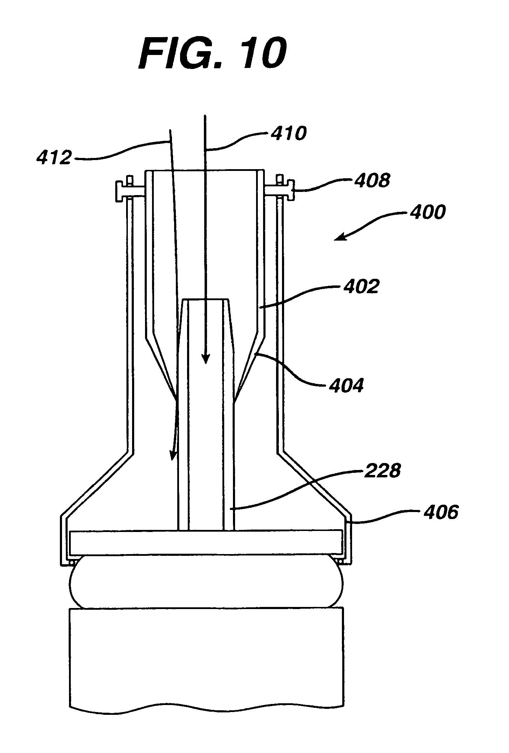

FIG. 10 illustrates a further concept of a non-occluding connector 400 according to the present invention. Similar to the connector 300 of FIG. 4, the connector 400 comprises a body 402 and seal 404 held onto the connection 228 by spring clips 406 connected to the body 402 by pins 408. The body 402 is annular, having an internal diameter exceeding the external diameter of the connection 228, and the seal 404 is formed by the body 402 tapering distally to a smaller diameter sufficient to engage the connection 228. Preferably the seal 404 also thins distally. A first flow path 410 passes through the connection 228 and with increasing pressure, as from increased flow, the seal 404 separates from the connection 228 to create a second flow path 412 past the seal 404 and bathing the remainder of the outer surface of the connection 228.

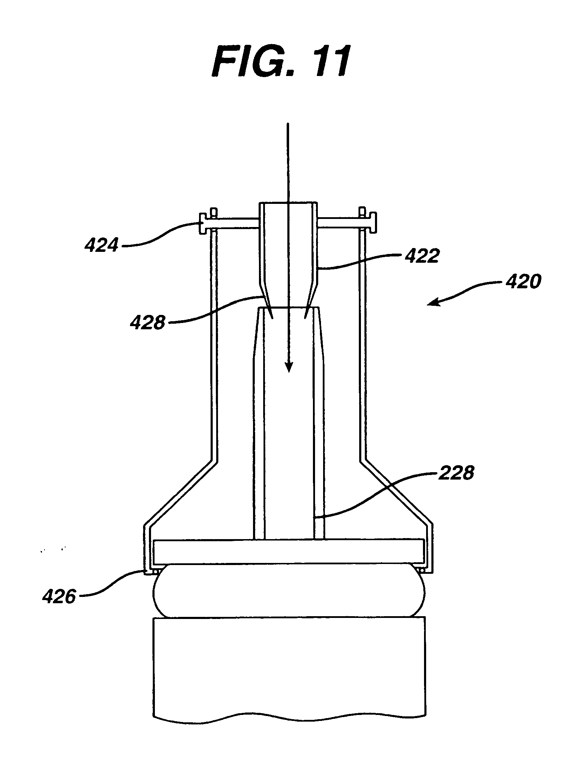

FIG. 11 illustrates a further connector 420 for the connection 228. It has an annular body 422, with pins 424 and clips 426 for attachment to the connection 228. The body 422 terminates in a distal seal 428 of similar shape to the seal 404. However, the body 422 and especially the seal 428 are of smaller diameter than in the previous embodiment. The seal 428 terminates in a diameter less than an internal diameter of the connection 228 and is disposed therein. Under low flows fluid passes between the seal 428 and the connection 228 as depicted in FIG. 11. Under stronger pressure, such as from increased flow through the connector 420, the seal 428 expands to engage the connection 228 and then directs all flow therethrough.

The cleaning and sterilization cycle in detail comprises the following steps.

### Step 1. Open the Lid

Pressing a foot pedal (not shown) opens the basin lid 16a. There is a separate foot pedal for each side. If pressure is removed from the foot pedal, the lid motion stops.

### Step 2. Position and connect the endoscope

The insertion tube 208 of the endoscope 200 is inserted into the helical circulation tube 64. The end section 224 and head section 202 of the endoscope 200 are situated within the basin 14a, with the feed hose 222 coiled within the basin 14a with as wide a diameter as possible.
The flush lines 30, preferably color-coded, are attached, one apiece, to the endoscope openings 226, 228, 228a, 230 and 232. The air line 112 is also connected to the connector 254. A guide located on the station 10 provides a reference for the color-coded connections.

### Step 3. Identify the user, endoscope, and specialist to the system

Depending on the customer-selectable configuration, the control system 20 may prompt for user code, patient ID, endoscope code, and/or specialist code. This information may be entered manually (through the touch screen) or automatically such as by using an attached barcode wand (not shown).

### Step 4. Close the basin lid

Closing the lid 16a preferably requires the user to press a hardware button and a touch-screen 22 button simultaneously (not shown) to provide a fail-safe mechanism for preventing the user's hands from being caught or pinched by the closing basin lid 16a. If either the hardware button or software button is released while the lid 16a is in the process of closing the motion stops.

### Step 5. Start Program

The user presses a touch-screen 22 button to begin the washing / disinfection process.

### Step 6. Pressurize the endoscope body and Measure the Leak Rate

The air pump is started and pressure within the endoscope body is monitored.
When pressure reaches 250 mbar, the pump is stopped, and the pressure is allowed to stabilize for 6 seconds. If pressure has not reached 250 mbar in 45 seconds the program is stopped and the user is notified of the leak. If pressure drops to less than 100 mbar during the 6-second stabilization period, the program is stopped and the user is notified of the condition.
Once the pressure has stabilized, the pressure drop is monitored over the course of 60 seconds. If pressure drops more than 10 mbar within 60 seconds, the program is stopped and the user is notified of the condition. If the pressure drop is less than 10 mbar in 60 seconds, the system continues with the next step. A slight positive pressure is held within the endoscope body during the rest of the process to prevent fluids from leaking in.

### Step 7. Check Connections

A second leak test checks the adequacy of connection to the various ports 226, 228, 228a, 230, 232 and the proper placement of the channel separator 240. A quantity of water is admitted to the basin 14a so as to submerge the distal end of the endoscope in the helical tube 64. Valve S1 is closed and valve S7 opened and the pumps 32 are run in reverse to draw a vacuum and to ultimately draw liquid into the endoscope channels 210 and 212. The pressure sensors 42 can be monitored to make sure that the pressure in any one channel does not drop by more than a predetermined amount in a given time frame. If it does, it likely indicates that one of the connections was not made correctly and air is leaking into the channel. In any event, in the presence of an unacceptable pressure drop the control system 20 will cancel the cycle and indicate a likely faulty connection, preferably with an indication of which channel failed. The volume of liquid which is drawn into the metering tube 136 in a given amount of time is measured and compared against a known standard for that particular endoscope model and channel. If the volume varies from the standard amount it indicates a failure. If the connection to the port 226 etc. is not tight, air will leak in and prevent sufficient volume entering the metering tube 136. Similarly, an obstruction within the endoscope channel will prevent sufficient volume entering the metering tube 136.

### PRE-RINSE

The purpose of this step is to flush water through the channels to remove waste material prior to washing and disinfecting the endoscope 200.

### Step 8. Fill basin

The basin 14a is filled with filtered water and the water level is detected by the pressure sensor 59 below the basin 14a.

### Step 9. Pump water through channels

The water is pumped via the pumps 32 through the interior of the channels 213, 214, 217, 218, 210 and 212 directly to the drain 74. This water is not recirculated around the exterior surfaces of the endoscope 200 during this stage.

### Step 10. Drain

As the water is being pumped through the channels, the drain pump 72 is activated to ensure that the basin 14a is also emptied. The drain pump 72 will be turned off when the drain switch 76 detects that the drain process is complete.

### Step 11. Blow air through channels

During the drain process sterile air is blown via the air pump 38 through all endoscope channels simultaneously to minimize potential carryover.

### WASH

### Step 12. Fill basin

The basin 14a is filled with warm water (35°C). Water temperature is controlled by controlling the mix of heated and unheated water. The water level is detected by the pressure sensor 59.

### Step 13. Add detergent

The system adds enzymatic detergent to the water circulating in the system by means of the peristaltic metering pump 88. The volume is controlled by controlling the delivery time, pump speed, and inner diameter of the peristaltic pump tubing.

### Step 14. Circulate wash solution

The detergent solution is actively pumped throughout the internal channels and over the surface of the endoscope 200 for a predetermined time period, typically of from one to five minutes, preferably about three minutes, by the channel pumps 32 and the external circulation pump 70. The inline heater 80 keeps the temperature at about 35 °C.

### Step 15. Start block test

After the detergent solution has been circulating for a couple of minutes, the flow rate through the channels is measured. If the flow rate through any channel is less than a predetermined rate for that channel, the channel is identified as blocked, the program is stopped, and the user is notified of the condition. The peristaltic pumps 32 are run at their predetermined flow rates and cycle off in the presence of unacceptably high pressure readings at the associated pressure sensor 42. If a channel is blocked the predetermined flow rate will trigger the pressure sensor 42 indicating the inability to adequately pass this flow rate. As the pumps 32 are peristaltic, their operating flow rate combined with the percentage of time they are cycled off due to pressure will provide the actual flow rate. The flow rate can also be estimated based upon the decay of the pressure from the time the pump 32 cycles off.

### Step 16. Drain

The drain pump 72 is activated to remove the detergent solution from the basin 14a and the channels. The drain pump 72 turns off when the drain level sensor 76 indicates that drainage is complete.

### Step 17. Blow air

During the drain process sterile air is blown through all endoscope channels simultaneously to minimize potential carryover.

### RINSE

### Step 18. Fill basin

The basin 14a is filled with warm water (35 °C). Water temperature is controlled by controlling the mix of heated and unheated water. The water level is detected by the pressure sensor 59.

### Step 19. Rinse

The rinse water is circulated within the endoscope channels (via the channel pumps 32) and over the exterior of the endoscope 200 (via the circulation pump 70 and the sprinkler arm 60) for 1 minute.

### Step 20. Continue Block test

As rinse water is pumped through the channels, the flow rate through the channels is measured and if it falls below the predetermined rate for any given channel, the channel is identified as blocked, the program is stopped, and the user is notified of the condition.

### Step 21. Drain

The drain pump is activated to remove the rinse water from the basin and the channels.

### Step 22. Blow air

During the drain process sterile air is blown through all endoscope channels simultaneously to minimize potential carryover.

### Step 23. Repeat rinse

Steps 18 through 22 are repeated to ensure maximum rinsing of enzymatic detergent solution from the surfaces of the endoscope and the basin.

### DISINFECT

### Step 24. Fill basin

The basin 14a is filled with very warm water (53 °C). Water temperature is controlled by controlling the mix of heated and unheated water. The water level is detected by the pressure sensor 59. During the filling process, the channel pumps 32 are off in order to ensure that the disinfectant in the basin is at the in-use concentration prior to circulating through the channels.

### Step 25. Add disinfectant

A measured volume of disinfectant 92, preferably CIDEX OPA orthophalaldehyde concentrate solution, available from Advanced Sterilization Products division Ethicon, Inc., Irvine, CA, is drawn from the disinfectant metering tube 96 and delivered into the water in the basin 14a via the metering pump 100. The disinfectant volume is controlled by the positioning of the fill sensor 98 relative to the bottom of the dispensing tube. The metering tube 96 is filled until the upper level switch detects liquid. Disinfectant 92 is drawn from the metering tube 96 until the level of the disinfectant in the metering tube is just below the tip of the dispensing tube. After the necessary volume is dispensed, the metering tube 96 is refilled from the bottle of disinfectant 92. Disinfectant is not added until the basin is filled, so that in case of a water supply problem, concentrated disinfectant is not left on the endoscope with no water to rinse it. While the disinfectant is being added, the channel pumps 32 are off in order to insure that the disinfectant in the basin is at the in-use concentration prior to circulating through the channels.

### Step 26. Disinfect

The in-use disinfectant solution is actively pumped throughout the internal channels and over the surface of the endoscope, ideally for a minimum of 5 minutes, by the channel pumps and the external circulation pump. The temperature is controlled by the in-line heater 80 to about 52.5 °C.

### Step 27. Flow check

During the disinfection process, flow through each endoscope channel is verified by timing the delivering a measured quantity of solution through the channel. Valve S1 is shut, and valve S7 opened, and in turn each channel pump 32 delivers a predetermined volume to its associated channel from the metering tube 136. This volume and the time it takes to deliver provides a very accurate flow rate through the channel. Anomalies in the flow rate from what is expected for a channel of that diameter and length are flagged by the control system 20 and the process stopped.

### Step 28. Continue Block Test

As disinfectant in-use solution is pumped through the channels, the flow rate through the channels is also measured as in Step 15.

### Step 29. Drain

The drain pump 72 is activated to remove the disinfectant solution from the basin and the channels.

### Step 30. Blow air

During the drain process sterile air is blown through all endoscope channels simultaneously to minimize potential carryover.

### FINAL RINSE

### Step 31. Fill basin

The basin is filled with sterile warm water (45 °C ) that has been passed through a 0.2 µ filter.

### Step 32. Rinse

The rinse water is circulated within the endoscope channels (via the channel pumps 32) and over the exterior of the endoscope (via the circulation pump 70 and the sprinkler arm 60) for 1 minute.

### Step 33. Continue Block test

As rinse water is pumped through the channels, the flow rate through the channels is measured as in Step 15.

### Step 34. Drain

The drain pump 72 is activated to remove the rinse water from the basin and the channels.

### Step 35. Blow air

During the drain process sterile air is blown through all endoscope channels simultaneously to minimize potential carryover.

### Step 36. Repeat rinse

Steps 31 through 35 are repeated two more times (a total of 3 post-disinfection rinses) to ensure maximum reduction of disinfectant residuals from the endoscope 200 and surfaces of the reprocessor.

### FINAL LEAK TEST

### Step 37. Pressurize the endoscope body and measure leak rate

Repeat Step 6.

### Step 38. Indicate program completion

The successful completion of the program is indicated on the touch screen.

### Step 39. De-pressurize the endoscope

From the time of program completion to the time at which the lid is opened, pressure within the endoscope body is normalized to atmospheric pressure by opening the vent valve S5 for 10 seconds every minute.

### Step 40. Identify the user

Depending on customer-selected configuration, the system will prevent the lid from being opened until a valid user identification code is entered.

### Step 41. Store program information

Information about the completed program, including the user ID, endoscope ID, specialist ID, and patient ID are stored along with the sensor data obtained throughout the program.

### Step 42. Print program record

If a printer is connected to the system, and if requested by the user, a record of the disinfection program will be printed.

### Step 43. Remove the endoscope

Once a valid user identification code has been entered, the lid may be opened (using the foot pedal as in step 1, above). The endoscope is then disconnected from the flush lines 30 and removed from the basin 14a. The lid can then be closed using both the hardware and software buttons as described in step 4, above.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A connector (300; 322; 342; 354; 400; 420) for connecting a lumen in a lumen device (200) to a source of fluid in an endoscope reprocessor, the connector comprising;
a coupling configured to engage with a port (228; 228a; 230; 232) connected to the lumen on the lumen device (200) wherein the port includes a first sealing surface (318);
a flow passage;
a sealing portion (304; 334; 346; 364; 404; 428) connected with the flow passage and comprising a second sealing surface shaped to engage the first sealing surface;
the sealing portion being formed of a resilient material; and
the sealing portion being adapted to engage with the first sealing surface under a condition of a first flow (314; 336; 348; 366; 410) in the connector;
**characterised by** the sealing portion (304; 334; 346; 364; 404; 428) being movably connected with the flow passage and being further adapted to disengage from the first sealing surface (318) under a condition of a second flow (316; 338; 350; 368; 412), different from the first flow (314; 336; 348; 366; 410), in the connector (300; 322; 342; 354; 400; 420).

2. A connector according to claim 1 wherein the resilient material of the sealing portion (304; 334; 346; 364; 404; 428) biases the second sealing surface into contact with the first sealing surface (318) when engaged therewith, the biasing being such that the second sealing surface seals against the first sealing surface when pressure within the flow passage associated with the first flow (314; 336; 348; 366; 410) is below a predetermined level preventing leakage of the flow there past and that the second surface does not seal against the first surface (318) when pressure within the flow passage associated with the second flow (316; 338; 350; 368; 412) is above the predetermined level thereby allowing flow over the first sealing surface (318).

3. A connector according to claim 1 wherein the resilient material of the sealing portion (304; 334; 346; 364; 404; 428) biases the second sealing surface away from contact with the first sealing surface (318) when engaged therewith, the biasing being such that the second sealing surface does not seal against the first sealing surface when pressure within the flow passage associated with the first flow (314; 336; 348; 366; 410) is below a predetermined level allowing leakage of the flow there past and wherein the sealing portion (304; 334; 346; 364; 404; 428) is oriented such that when pressure within the flow passage associated with the second flow (316; 338; 350; 368; 412) is above the predetermined level, such pressure urges the second sealing surface into contact with the first sealing surface (318) thereby preventing leakage of the flow there past.

4. A connector according to claim 1 wherein the sealing portion (304; 334; 346; 364; 404; 428) is formed integral with the connector (300; 322; 342; 354; 400; 420).

5. A connector according to claim 1 wherein the first sealing surface (318) is cylindrical and wherein the sealing portion (304; 334; 346; 364; 404; 428) comprises an annular flange sized to seat against the first sealing surface (318).

6. A connector according to claim 1 wherein the sealing portion (304; 334; 346; 364; 404; 428) comprises a free distal edge.

7. A connector according to claim 1 wherein the sealing portion (304; 334; 346; 364; 404; 428) is formed of silicone.

8. A connector according to claim 1 wherein the first flow (314; 336; 348; 366; 410) is associated with a pressure at the sealing portion less than 0.83 bar (12 psig) and the second flow (316; 338; 350; 368; 412) is associated with a pressure at the sealing portion greater than 0.83 bar (12 psig).

9. A connector according to claim 1 wherein the first flow (314; 336; 348; 366; 410) is associated with a pressure at the sealing portion (304; 334; 346; 364; 404; 428) greater than 035 bar (5 psig) and the second flow (316; 338; 350; 368; 412) is associated with a pressure at the sealing portion less than 035 bar (5 psig).

10. A method of connecting a port (228; 228a; 230; 232) on a lumen device (200) to an endoscope reprocessor and flowing fluid through the port; the method comprising the steps of:
attaching a coupling on the endoscope reprocessor to the port (228; 228a; 230; 232), the coupling having a flow passage therethrough and a sealing portion (304; 334; 346; 364; 404; 428) attached to the flow passage, and being formed of a resilient material;
flowing a first flow (314; 336; 348; 366; 410) through the flow passage and into the port (228; 228a; 230; 232);
**characterised by**:
the sealing portion (304; 334; 346; 364; 404; 428) being movably attached to the flow passage and being moveable from a first position to a second position;
the resilient material biasing the sealing portion (304; 334; 346; 364; 404; 428) into one of the first position or second position;
the step of flowing a first flow (314; 336; 348; 366; 410) causing the sealing portion (304; 334; 346; 364; 404; 428) to move into the first position;
the method further including the step of flowing a second, different, flow (316; 338; 350; 368; 412) through the flow passage causing the sealing portion (304; 334; 346; 364; 404; 428) to move into the second position; and
wherein one of the first position and the second position comprises a sealing position in which the sealing portion (304; 334; 346; 364; 404; 428) seals against a first sealing surface (318) on the port (228; 228a; 230; 232) to prevent flow there past, and wherein the other of the first position and the second position comprises a non-sealing position in which the sealing portion (304; 334; 346; 364; 404; 428) is away from the first sealing surface (318) to allow flow over the first sealing surface (318).

11. A method according to claim 10 wherein the first position is the scaling position.

12. A method according to claim 11 wherein the sealing portion (304; 334; 346; 364; 404; 428) is biased toward the first position.

13. A method according to claim 10 wherein the sealing portion (304; 334; 346; 364; 404; 428) is biased toward the first position.

14. A method according to claim 10 wherein the second flow (316; 338; 350; 368; 412) has a higher pressure than the first flow (314; 336; 348; 366; 410).

15. A method according to claim 14 wherein the higher pressure is created by increasing volume of the second flow (316; 338; 350; 368; 412).

16. A method according to claim 14 wherein the second flow (316; 338; 350; 368; 412) has a pressure above 0.83 bar (12 psig).

17. A method according to claim 10 wherein the second flow (316; 338; 350; 368; 412) is in a different direction than the first flow (314; 336; 348; 366; 410).

18. A method according to claim 10 wherein the first flow (314; 336; 348; 366; 410) comprises a fluid selected from a list consisting of: a washing fluid, an antimicrobial agent, and combinations thereof.

## Patentansprüche

1. Verbinder (300; 322; 342; 354; 400; 420) zum Verbinden eines Lumens in einer Lumenvorrichtung (200) mit einer Fluidquelle in einem Endoskop-Reprozessor, wobei der Verbinder aufweist:
eine Kopplung, die so ausgestaltet ist, dass sie an einen Port (228; 228a; 230; 232) anschließt, der mit dem Lumen auf der Lumenvorrichtung (200) verbunden ist, wobei der Port eine erste Dichtfläche (318) umfasst;
einen Strömungsdurchlass;
einen Dichtbereich (304; 334; 346; 364; 404; 428), der mit dem Strömungsdurchlass verbunden ist und eine zweite Dichtfläche aufweist, die so geformt ist, dass sie an der ersten Dichtfläche anliegt;
wobei der Dichtbereich aus einem nachgiebigen Material gebildet ist; und
wobei der Dichtbereich so ausgelegt ist, dass er unter der Bedingung einer ersten Strömung (314; 416, 348; 366; 410) in dem Verbinder an der ersten Dichtfläche anliegt;
**dadurch gekennzeichnet, dass** der Dichtbereich (304; 334; 346; 364; 404; 428) beweglich mit dem Strömungsdurchlass verbunden ist und weiter so ausgelegt ist, dass er sich von der ersten Dichtfläche (318) unter der Bedingung einer zweiten Strömung (316; 338; 350; 368; 412) löst, die von der ersten Strömung (314; 416, 348; 366; 410) in dem ersten Verbinder (300; 322; 342; 354; 400; 420) unterschiedlich ist.

2. Verbinder nach Anspruch 1, bei dem das nachgiebige Material des Dichtbereiches (304; 334; 346; 364; 404; 428) die zweite Dichtfläche in den Kontakt mit der ersten Dichtfläche (318) vorbelastet, wenn sie daran anliegt, wobei die Vorbelastung derart ist, dass die zweite Dichtfläche gegen die erste Dichtfläche abdichtet, wenn der Druck innerhalb des Strömungsdurchlasses, der mit der ersten Strömung (314; 416, 348; 366; 410) verknüpft ist, unterhalb eines vorbestimmten Wertes liegt, um ein Entweichen der Strömung an diesen vorbei zu verhindern, und dass die zweite Fläche nicht gegen die erste Fläche (318) abdichtet, wenn der Druck innerhalb des Strömungsdurchlasses, der mit der zweiten Strömung (316; 338; 350; 368; 412) verknüpft ist, oberhalb des vorbestimmten Wertes liegt, so dass eine Strömung über die erste Dichtfläche (318) erlaubt wird.

3. Verbinder nach Anspruch 1, bei dem das nachgiebige Material des Dichtbereiches (304; 334; 346; 364; 404; 428) die zweite Dichtfläche weg aus dem Kontakt mit der ersten Dichtfläche (318) vorbelastet, wenn sie daran anliegt, wobei die Vorbelastung derart ist, dass die zweite Dichtfläche nicht gegen die erste Dichtfläche abdichtet, wenn der Druck innerhalb des Strömungsdurchlasses, der mit der ersten Strömung (314; 416, 348; 366; 410) verbunden ist, unterhalb eines vorbestimmten Wertes liegt, der das Entweichen der Strömung an diesen vorbei erlaubt, und bei dem der Dichtbereich (304; 334; 346; 364; 404; 428) so ausgerichtet ist, dass, wenn der Druck innerhalb des Strömungsdurchlasses, der mit der zweiten Strömung (316; 338; 350; 368; 412) verknüpft ist, oberhalb des vorbestimmten Wertes ist, ein solcher Druck die zweite Dichtfläche in den Kontakt mit der ersten Dichtfläche (318) zwingt, so dass das Entweichen der Strömung an diesen vorbei verhindert wird.

4. Verbinder nach Anspruch 1, bei dem der Dichtbereich (304; 334; 346; 364; 404; 428) einstückig mit dem Verbinder (300; 322; 342; 354; 400; 420) ausgebildet ist.

5. Verbinder nach Anspruch 1, bei dem die erste Dichtfläche (318) zylindrisch ist und bei dem der Dichtbereich (304; 334; 346; 364; 404; 428) einen ringförmigen Flansch aufweist, der so bemessen ist, dass er gegen die erste Dichtfläche (318) anliegt.

6. Verbinder nach Anspruch 1, bei dem der Dichtbereich (304; 334; 346; 364; 404; 428) eine freie distale Kante aufweist.

7. Verbinder nach Anspruch 1, bei dem der Dichtbereich (304; 334; 346; 364; 404; 428) aus Silikon gebildet ist.

8. Verbinder nach Anspruch 1, bei dem die erste Strömung (314; 416, 348; 366; 410) mit einem Druck am Dichtbereich von weniger als 0.83 bar (12 psig) verknüpft ist und die zweite Strömung (316; 338; 350; 368; 412) mit einem Druck am Dichtbereich größer als 0.83 bar (12 psig) verknüpft ist.

9. Verbinder nach Anspruch 1, bei dem die erste Strömung (314; 416, 348; 366; 410) mit einem Druck am Dichtbereich von größer als 0.35 bar (5 psig) verknüpft ist und die zweite Strömung (316; 338; 350; 368; 412) mit einem Druck am Dichtbereich kleiner als 0.35 bar (5 psig) verknüpft ist.

10. Verfahren zum Verbinden eines Ports (228; 228a; 230; 232) auf einer Lumenvorrichtung (200) mit einem Endoskop-Reprozessor und zum Leiten von Fluid durch den Port; wobei das Verfahren die Schritte aufweist:
Befestigen einer Kopplung an dem Endoskop-Reprozessor an dem Port (228; 228a; 230; 232), wobei die Kopplung einen durchgehenden Strömungsdurchlass und einen Dichtbereich (304; 334; 346; 364; 404; 428) hat, der an dem Strömungsdurchlass befestigt ist und aus einem nachgiebigen Material gebildet ist;
Leiten einer ersten Strömung (314; 416, 348; 366; 410) durch den Strömungsdurchlass in den Port (228; 228a; 230; 232);
**dadurch gekennzeichnet, dass**:
der Dichtbereich (304; 334; 346; 364; 404; 428) bewegbar an dem Strömungsdurchlass befestigt ist und aus einer ersten Position in eine zweite Position bewegbar ist;
das nachgiebige Material den Dichtbereich (304; 334; 346; 364; 404; 428) in entweder die erste Position oder die zweite Position vorbelastet;
wobei der Schritt des Leitens einer ersten Strömung (314; 416, 348; 366; 410) bewirkt, dass der Dichtbereich (304; 334; 346; 364; 404; 428) sich in die erste Position bewegt;
wobei das Verfahren weiter den Schritt des Leitens einer zweiten unterschiedlichen Strömung (316; 338; 350; 368; 412) durch den Strömungsdurchlass umfasst, was bewirkt, dass sich der Dichtbereich (304; 334; 346; 364; 404; 428) in die zweite Position bewegt; und
wobei entweder die erste Position oder die zweite Position eine Dichtposition aufweist, in der der Dichtbereich (304; 334; 346; 364; 404; 428) gegen eine erste Dichtfläche (318) auf dem Port (228; 228a; 230; 232) abdichtet, um die Strömung an dieser vorbei zu verhindern, und wobei die andere, die erste oder die zweite Position, eine Nicht-Dichtposition aufweist, in der der Dichtbereich (304; 334; 346; 364; 404; 428) sich entfernt von der ersten Dichtfläche (318) befindet, um das Strömen über die erste Dichtfläche (318) zu erlauben.

11. Verfahren nach Anspruch 10, bei dem die erste Position die Dichtposition ist.

12. Verfahren nach Anspruch 11, bei dem der Dichtbereich (304; 334; 346; 364; 404; 428) auf die erste Position zu belastet wird.

13. Verfahren nach Anspruch 10, bei dem der Dichtbereich (304; 334; 346; 364; 404; 428) auf die erste Position zu belastet wird.

14. Verfahren nach Anspruch 10, bei dem die zweite Strömung (316; 338; 350; 368; 412) einen höheren Druck hat als die erste Strömung (314; 416, 348; 366; 410).

15. Verfahren nach Anspruch 14, bei dem der höhere Druck erzeugt wird, indem das Volumen der zweiten Strömung (316; 338; 350; 368; 412) erhöht wird.

16. Verfahren nach Anspruch 14, bei dem die zweite Strömung (316; 338; 350; 368; 412) einen Druck über 0.83 bar (12 psig) hat.

17. Verfahren nach Anspruch 10, bei dem die zweite Strömung (316; 338; 350; 368; 412) in eine von der ersten Strömung (314; 416, 348; 366; 410) unterschiedliche Richtung läuft.

18. Verfahren nach Anspruch 10, bei dem die erste Strömung (314; 416, 348; 366; 410) ein Fluid aufweist, das aus einer Liste bestehend aus: einem Waschfluid, einer Antimikrobiellen Substanz und Kombinationen aus diesen ausgewählt ist.

## Revendications

1. Raccord (300 ; 322 ; 342 ; 354 ; 400 ; 420) pour relier une lumière dans un dispositif de lumière (200) à une source de fluide dans un dispositif de retraitement pour endoscopes, le raccord comprenant :
■ un manchon configuré pour se mettre en prise avec un orifice (228 ; 228a ; 230 ; 232) relié à la lumière sur le dispositif de lumière (200), dans lequel l'orifice comprend une première surface d'étanchéité (318) ;
■ un passage d'écoulement ;
■ une partie d'étanchéité (304 ; 334 ; 346 ; 364 ; 404 ; 428) reliée au passage d'écoulement et comprenant une seconde surface d'étanchéité façonnée pour se mettre en prise avec la première surface d'étanchéité ;
■ la partie d'étanchéité étant formée d'un matériau élastique ; et
■ la partie d'étanchéité étant adaptée pour se mettre en prise avec la première surface d'étanchéité dans une condition d'un premier écoulement (314 ; 336 ; 348 ; 366 ; 410) dans le raccord ;
**caractérisé en ce que** la partie d'étanchéité (304 ; 334 ; 346 ; 364 ; 404 ; 428) est reliée de façon mobile au passage d'écoulement et est en outre adaptée pour se dégager de la première surface d'étanchéité (318) dans une condition d'un second écoulement (316 ; 338 ; 350 ; 368 ; 412), différente du premier écoulement (314 ; 336 ; 348 ; 366 ; 410) dans le raccord (300 ; 322 ; 342 ; 354 ; 400 ; 420).

2. Raccord selon la revendication 1, dans lequel le matériau élastique de la partie d'étanchéité (304 ; 334 ; 346 ; 364 ; 404 ; 428) force la seconde surface d'étanchéité en contact avec la première surface d'étanchéité (318) lorsqu'elle est en prise avec elle, la force étant telle que la seconde surface d'étanchéité se scelle contre la première surface d'étanchéité lorsque la pression à l'intérieur du passage d'écoulement associée au premier écoulement (314 ; 336 ; 348 ; 366 ; 410) est inférieure à un niveau prédéterminé empêchant une fuite de l'écoulement au-delà de celle-ci et telle que la seconde surface ne vient pas se sceller contre la première surface (318) lorsque la pression à l'intérieur du passage d'écoulement associé au second écoulement (316 ; 338 ; 350 ; 368 ; 412) est supérieure au niveau prédéterminé, permettant ainsi un écoulement par dessus la première surface d'étanchéité (318).

3. Raccord selon la revendication 1, dans lequel le matériau élastique de la partie d'étanchéité (304 ; 334 ; 346 ; 364 ; 404 ; 428) écarte la seconde surface d'étanchéité du contact avec la première surface d'étanchéité (318) lorsqu'elle est en prise avec elle, la force d'écartement étant telle que la seconde surface d'étanchéité ne se scelle pas contre la première surface d'étanchéité lorsque la pression à l'intérieur du passage d'écoulement associé au premier écoulement (314 ; 336 ; 348 ; 366 ; 410) est inférieure à un niveau prédéterminé, permettant la fuite de l'écoulement au-delà de celle-ci et dans lequel la partie d'étanchéité (304 ; 334 ; 346 ; 364 ; 404 ; 428) est orientée de telle sorte que lorsque la pression à l'intérieur du passage d'écoulement associé au second écoulement (316 ; 338 ; 350 ; 368 ; 412) est supérieure au niveau prédéterminé, une telle pression presse la seconde surface d'étanchéité en contact avec la première surface d'étanchéité (318), empêchant ainsi une fuite de l'écoulement au-delà de celle-ci.

4. Raccord selon la revendication 1, dans lequel la partie d'étanchéité (304 ; 334 ; 346 ; 364 ; 404 ; 428) est formée d'un seul tenant avec le raccord (300 ; 322 ; 342 ; 354 ; 400 ; 420).

5. Raccord selon la revendication 1, dans lequel la première surface d'étanchéité (318) est cylindrique et dans lequel la partie d'étanchéité (304 ; 334 ; 346 ; 364 ; 404 ; 428) comprend un rebord annulaire dimensionné pour reposer contre la première surface d'étanchéité (318).

6. Raccord selon la revendication 1, dans lequel la partie d'étanchéité (304 ; 334 ; 346 ; 364 ; 404 ; 428) comprend un premier bord distal.

7. Raccord selon la revendication 1, dans lequel la partie d'étanchéité (304 ; 334 ; 346 ; 364 ; 404 ; 428) est formée de silicone.

8. Raccord selon la revendication 1, dans lequel le premier écoulement (314 ; 336 ; 348 ; 366 ; 410) est associé à une pression au niveau de la partie d'étanchéité inférieure à 0,83 bar (12 psig) et le second écoulement (316 ; 338 ; 350 ; 368 ; 412) est associé à une pression au niveau de la partie d'étanchéité supérieure à 0,83 bar (12 psig).

9. Raccord selon la revendication 1, dans lequel le premier écoulement (314 ; 336 ; 348 ; 366 ; 410) est associé à une pression au niveau de la partie d'étanchéité (304 ; 334 ; 346 ; 364 ; 404 ; 428) supérieure à 0,35 bar (5 psig) et le second écoulement (316 ; 338 ; 350 ; 368 ; 412) est associé à une pression au niveau de la partie d'étanchéité inférieure à 0,35 bar (5 psig).

10. Procédé de raccordement d'un orifice (228 ; 228a ; 230 ; 232) sur un dispositif de lumière (200) à un dispositif de retraitement pour endoscopes et d'écoulement de fluide à travers l'orifice ; le procédé comprenant les étapes consistant à :
■ fixer un manchon sur le dispositif de retraitement pour endoscopes à l'orifice (228 ; 228a ; 230 ; 232), le manchon étant traversé par un passage d'écoulement et ayant une partie d'étanchéité (304 ; 334 ; 346 ; 364 ; 404 ; 428) fixée au passage d'écoulement et formée d'un matériau élastique ;
■ faire passer un premier écoulement (314 ; 336 ; 348 ; 366 ; 410) à travers le passage d'écoulement et dans l'orifice (228 ; 228a ; 230 ; 232) ;
**caractérisé en ce que** :
■ la partie d'étanchéité (304 ; 334 ; 346 ; 364 ; 404 ; 428) est fixée de façon mobile au passage d'écoulement et est mobile d'une première position à une seconde position ;
■ le matériau élastique amène la partie d'étanchéité (304 ; 334 ; 346 ; 364 ; 404 ; 428) dans une de la première ou de la seconde position ;
■ l'étape de passage d'un premier écoulement (314 ; 336 ; 348 ; 366 ; 410) entraîne le déplacement de la partie d'étanchéité (304 ; 334 ; 346 ; 364 ; 404 ; 428) dans la première position ;
■ le procédé comprenant en outre l'étape de passage d'un second écoulement différent (316 ; 338 ; 350 ; 368 ; 412) à travers le passage d'écoulement, entraînant le déplacement de la partie d'étanchéité (304 ; 334 ; 346 ; 364 ; 404 ; 428) dans la seconde position ; et
■ dans lequel une de la première position et de la seconde position comprend une position d'étanchéité dans laquelle la partie d'étanchéité (304 ; 334 ; 346 ; 364 ; 404 ; 428) repose contre une première surface d'étanchéité (318) sur l'orifice (228 ; 228a ; 230 ; 232) pour empêcher un écoulement au-delà de celle-ci, et dans lequel l'autre de la première position et de la seconde position comprend une position de non-étanchéité dans laquelle la partie d'étanchéité (304 ; 334 ; 346 ; 364 ; 404 ; 428) est à l'écart de la première surface d'étanchéité (318) pour permettre un écoulement par dessus de la première surface d'étanchéité (318).

11. Procédé selon la revendication 10, dans lequel la première position est la position d'étanchéité.

12. Procédé selon la revendication 11, dans lequel la partie d'étanchéité (304 ; 334 ; 346 ; 364 ; 404 ; 428) est amenée vers la première position.

13. Procédé selon la revendication 10, dans lequel la partie d'étanchéité (304 ; 334 ; 346 ; 364 ; 404 ; 428) est amenée vers la première position.

14. Procédé selon la revendication 10, dans lequel le second écoulement (316 ; 338 ; 350 ; 368 ; 412) a une pression supérieure à celle du premier écoulement (314 ; 336 ; 348 ; 366 ; 410).

15. Procédé selon la revendication 14, dans lequel la pression plus élevée est créée en augmentant le volume du second écoulement (316 ; 338 ; 350 ; 368 ; 412).

16. Procédé selon la revendication 14, dans lequel le second écoulement (316 ; 338 ; 350 ; 368 ; 412) a une pression supérieure à 0,83 bar (12 psig).

17. Procédé selon la revendication 10, dans lequel le second écoulement (316 ; 338 ; 350 ; 368 ; 412) est dans une direction différente de celle du premier écoulement (314 ; 336 ; 348 ; 366 ; 410).

18. Procédé selon la revendication 10, dans lequel le premier écoulement (314 ; 336 ; 348 ; 366 ; 410) comprend un fluide choisi dans une liste comprenant : un liquide de lavage, un agent antimicrobien et des combinaisons de ceux-ci.
